# EUROPEAN PATENT SPECIFICATION

(11) **EP 1 181 371 B1**
(45) Date of publication and mention of the grant of the patent: **22.10.2003**
(21) Application number: 00956156.4
(22) Date of filing: 02.06.2000
(51) Int. Cl.: C12N 15/31, C12N 1/21, C07K 14/245, A61K 39/108

(54) **ATTENUATED MUTANT ENTEROPATHOGENIC E. COLI (EPEC) STRAINS, PROCESS FOR THEIR PRODUCTION AND THEIR USE**
ATTENUIERTE MUTANTEN ENTEROPATHOGENISCHER E. COLI STÄMME (EPEC), VERFAHREN DEREN PRODUKTION UND VERWENDUNGEN
SOUCHES ATTENUEES MUTANTES D'E. COLI ENTEROPATHOGENE (EPEC), PROCEDE DE PRODUCTION ET UTILISATION

(30) Priority: 02.06.1999 EP 99870111
(43) Date of publication of application: 27.02.2002
(73) Proprietor: Centrum voor Onderzoek in Diergeneeskunde en Agrochemie, 1180 Brussel (Ukkel) (BE)
(72) Inventor: MARIEN, Jonas, B-3000 Leuven (BE); PEETERS, Johan, B-9320 Erembodegem (BE); VANDEKERCHOVE, Dominique, B-1560 Leuven (BE); STAKENBORG, Wim, B-3900 Overpelt (BE)
(74) Representative: De Clercq, Ann
(86) International application number: EP0005061
(87) International publication number: WO00073462

(56) References cited:
- EP-A- 0 080 806
- NOUGAYR DE J.-P. ET AL.: "The long-term cytoskeletal rearrangement induced by rabbit enteropathogenic Escherichia coli is Esp dependent but intimin independent" MOLECULAR MICROBIOLOGY, vol. 31, no. 1, January 1999 (1999-01), pages 19-30, XP000960505
- DONNENBERG M.S. ET AL.: "Effect of a prior experimental human enteropathic Escherichia coli infection on illness following homologous and heterologous rechallenge" INFECT. IMMUN., vol. 66, no. 1, January 1998 (1998-01), pages 52-58, XP002121475
- DONNENBERG M.S. & KAPER J.B.: "Construction of an eae deletion mutant of enteropathogenic Escherichia coli by using a positive-selection suicide vector" INFECT. IMMUN., vol. 59, no. 12, December 1991 (1991-12), pages 4310-4317, XP002121476 cited in the application
- ADAMS L.M. ET AL.: "Identification and characterization of a K88- and CS31A-like operon of a rabbit enteropathogenic Escherichia coli strain which encodes fimbriae involved in the colonization of rabbit intestine" INFEC. IMMUN., vol. 65, no. 12, December 1997 (1997-12), pages 5222-5230, XP002154573

<110> CENTRUM VOOR ONDERZOEK IN DIERGENEESKUNDE EN AGROC
<120> ATTENUATED MUTANT ENTEROPATHOGENIC E. COLI (EPEC)
   STRAINS, PROCESS FOR THEIR PRODUCTION AND THEIR USE
<130> CODA-001-PCT

<140>
<141>

<160> 8
<170> PatentIn Ver. 2.1

<210> 1
<211> 31
<212> DNA
<213> Artificial Sequence

<220>
<223> Description of Artificial Sequence:primer

<400> 1
   aaaaaaggat ccaaaaatta cgccggaaga t 31

<210> 2
<211> 31
<212> DNA
<213> Artificial Sequence

<220>
<223> Description of Artificial Sequence:primer

<400> 2
   aaaaaatcta gaaaaaggta aackaggtct c 31

<210> 3
<211> 22
<212> DNA
<213> Artificial Sequence

<220>
<223> Description of Artificial Sequence:primer

<210> 4
<211> 21
<212> DNA
<213> Artificial Sequence

<220>
<223> Description of Artificial Sequence:primer

<400> 4
   tccagtgaac taccgtcaaa g 21

<210> 5
<211> 20
<212> DNA
<213> Artificial Sequence

<220>
<223> Description of Artificial Sequence:primer

<400> 5
   gaaactaatt gctattgctg 20

<210> 6
<211> 20
<212> DNA
<213> Artificial Sequence

<220>
<223> Description of Artificial Sequence:primer

<400> 6
   atgcgattga aatagttagc 20

<210> 7
<211> 20
<212> DNA
<213> Artificial Sequence

<220>
<223> Description of Artificial Sequence:primer

<210> 8
<211> 20
<212> DNA
<213> Artificial Sequence

<220>
<223> Description of Artificial Sequence:primer

<400> 8
   agcgcttttt gtttacttcc 20

## Description

The present invention concerns a process of obtaining attenuated mutant strains of enteropathogenic *Escherichia coli* (EPEC), derived from strains that carry partly or entirely the locus of enterocyte effacement, which supplies them with certain pathogenic features. These features are, among others, effacement of the intestinal villi or brush border, rearrangement of the cytoskelet structure and resulting diarrhea. The invention equally concerns the attenuated strains that are the result of this process and their use in a vaccine.

EPEC strains are known as an important etiologic agent of human infant and animal diarrhea by adhering to the intestinal mucosa and producing the characteristic attaching and effacing (AE) lesion in the brush border microvillous membrane (26). They are also the prototype for a family of pathogens exhibiting this unique virulence mechanism (27). This group of attaching and effacing (AE) *Escherichia coli* includes enterohemorrhagic *E.coli, Hafnia alvei* and the mouse pathogen *Citrobacter rodentium* (6, 22). EPEC strains are considered as the only important class of AE *E. coli* in rabbits (34). It was further demonstrated that rabbit EPEC type strain RDEC-1 possesses the same attributes as described for human EPEC strains (6, 17). Rabbit EPEC strains attach to intestinal tissue and cells and efface microvilli. Until present no known thermostable or thermolabile enterotoxins are described and the grand majority of these strains are not enteroinvasive (33a).

Next to the genetic features that can be more easily detected at present than before and the characteristic AE effects which are described more in detail further on, there are some additional characteristics which can be detected by using more traditional methods. Following the serological characterization of Salmonellae, the serotyping scheme for *Escherichia coli* strains was developed, originally based on three types of antigen: the somatic (O) antigen, the capsular (K) antigen and the flagellar (H) antigen. The somatic O antigens are lipopolysaccharide complexes and part of the cell wall structure of the *E. coli*. The polysaccharide repeating units give the O antigens their specific immunogenicity. A number of the O antigens cross-react serologically with or are even identical both chemically and serologically to somatic antigens of other organisms, like members of the *Shigella* and *Salmonella* groups. Some strains, called rough strains and designated OR, lack these repeating side chains and become auto-agglutinable. The capsular K antigens are mainly acidic polysaccharide. At current, K antigen typing is not regularly performed, except for some cases. The diversity of the flagellar H antigens is based on the different types of flagellin present as part of the flagellar structure. Many *E. coli* are either only slightly motile or non-motile on primary isolation. However, many strains on passage through a semisolid agar attain full motility. Those strains not developing motility are designated non-motile (NM) or H-. Thus this O:K:H type describes the serological features of *E. coli* in full. In general the K antigen is left off and a strain will be considered as serotyped by just detecting the O:H type. In some cases also fimbrial F antigens are detected and in that case full typing of a strain results in a O:K:H:F designation.

There are indications that some serotypes are more likely to be associated with certain microenvironments. However, serotyping just labels the organisms without showing their specific pathogenicity or virulence factors. Certain serotypes appear to be linked with certain virulence traits (42). Long before their virulence characteristics were known, EPEC were first demonstrated on the basis of their serotype. In rabbit, there are a number of serotypes described as being more (O15, O26 and O103) or less (O128, O132) pathogenic. There is also one serotype, O109 that seems to be more pathogenic with suckling rabbits and less effective against weaned rabbits. Thus, serotyping gives us, in combination with a biotyping scheme, a first indication on the virulent properties of isolated rabbit EPEC strains.

A biotyping scheme based on the ability of strains to ferment dulcitol, raffinose, rhamnose and sorbose and to decarboxylate omithine was shown to give optimal discrimination among rabbit EPEC strains. By applying this scheme, 21 biotypes could be established. With these biotypes, in combination with the serotype and motility of the strain, certain pathotypes can be distinguished. In general, strains that are designated O15:H-/3 (O15 positive, non-motile and belonging to biotype 3), O26:H+/4 and O103:H+/8 are to be considered as highly pathogenic for weaned rabbits. Other types like O132:H+/2 and O128:H+/2 include strains that have very different pathogenic features, from non-pathogenic to highly pathogenic but generally only modestly pathogenic. Contrary to the afore mentioned strains, the O109:H+/1 type is more often associated with diarrhea in suckling rabbits and can be highly pathogenic to these infantile rabbits. Although more types can be isolated from diarrheal rabbits, the above mentioned pathotypes are the most important and the most frequently isolated in case of EPEC associated diarrhea with rabbits.

Apart from these more classic techniques, much work has been done on revealing the underlying genetic properties of EPEC, specifically human isolates. All genes necessary for AE are encoded on a 35kb chromosomal pathogenicity island called the locus of enterocyte effacement (LEE) (22). Among others, genes encoding a type III secretion system, secreted proteins (Esp) and the adhesine intimin were characterized and are situated in this island (1, 2, 11, 15, 16). Experiments confirmed that a cloned EPEC LEE is able to introduce the AE phenotype in an *E.coli* K12 host. Similar pathogenicity islands are found in all other AE pathogens, including those strains isolated from diarrheal rabbits (22, 41).

The AE virulence mechanism is preceded by an initial loose adherence. This microbial attachment to host cells or tissue surfaces, mediated by the interaction of an adhesion molecule (i.e. adhesine) and a receptor molecule, is characterized by a high degree of specificity. Adhesines take several forms, most often as distinct morphological structures called fimbriae (also known as pill) and fibrils, but also as afimbrial surface proteins (7a). In case of EPEC's, some adhesines are well characterized but probably even more remain unknown. Although most adhesines are not necessary for AE, they certainly can increase a strain's virulence. For instance the EAF plasmid encoded virulence factor (Per) and bundle forming pili, which are believed to make a rapid colonization of the surrounding region possible, have a strongly positive effect on the virulence of human EPEC strains (14, 19). In certain strains intimin is the only known adhesine (13a, 17a, 21a), hence deletion will make adherence impossible. After an initial loose adherence, the recently described translocated intimin receptor (Tir) and certain Esp proteins are secreted by the type III secretion system (49). As a result, some proteins, including Tir, are transferred into the host cell cytoplasm (18, 32). Transfer of Tir and positioning of this receptor in the host cell membrane allows EPEC to adhere intimately by binding of intimin to this receptor.

Underneath the attached EPEC, cytoskeletal rearrangements that are induced by the attaching cell result in localised degeneration of the epithelial brush border and the AE lesion, also described as pedestal formation (9, 27, 28, 43). Resulting tissue damage leads to direct nutrient losses and a diminished food uptake.

The outer membrane protein intimin is transcribed from the *eae* gene and is considered as an important protein for the virulence mechanism. Mutants deffective in *eae* form immature AE lesions and do not induce reorganization of the cytoskeletal structure underneath the locus of infection (8). Immune response against the intimin protein is considered as a major immunoprotective factor against EPEC infection (8, 47). Deletion mutagenesis of *eae* in a human EPEC strain resulted in a strain that caused diarrhea in 4 of 11 of the volunteers that received this strain. Furthermore, an experimental infection later in time with a fully virulent EPEC strain of individuals that were experimentally vaccinated with the mutant strain demonstrated that application of such a vaccine failed to induce a long-term protection (8, 10).

The present inventors have extensively studied EPEC as a causative agent of diarrhea in weaned and suckling rabbits. EPEC strains were identified as an important factor in rabbit enteropathy (39). Pathogenic properties and the absence of thermostable or thermolabile enterotoxins were described (33a). Experimental infections with strains isolated from newborn, suckling rabbits and from weaned rabbits were performed and clinical studies revealed findings that were similar to the ones described in human EPEC infections (33, 35).

At present, there is only one solution for a rabbit breeder when colibacillosis arises: treating the animals with antibiotics and eventually eliminating the animals and repopulating the farm. However, antibiotic resistance is increasing and no guarantee is given that new animals are completely free of EPEC strains. No vaccines that are both safe and effective are currently available. Some experiments have been performed on using formalin-killed strains as possible vaccines against infection with O103 strains (5, 23), without directly useful results. Also naturally isolated strains that carried only some antigens of the pathogenic O103 serogroup have been tested as a vaccine, still without a safe protection against infection with a wild-type strain (25). More recently, the use of a strain mutated in the *sep* region (responsible for the type III secretion system) has been described (4a). Good results were obtained, but the strain was attenuated by using transposon mutagenesis and carries a kanamycin resistance gene, which makes it unsuitable for practical use. The strain, its method of obtaining and the eventual use of it has been described in a French patent application published under number 2 758 568 (24). In a more recent publication, the same researchers report the construction of similar strains, mutated in *espA, espB, espD* and *eae* respectively (29). As been reported before, strains mutated this way did not induce attaching and effacing (1, 2, 8, 10, 20, 45). In the *eae* mutant O103 strain, a slow cytopathic effect (CPE) was still noticed. This effect was abolished in the *esp* mutants and in earlier reported AF/R2 mutants. AF/R2 is an adhesine that has been reported before and for which there is a homologue present in O15:H- strains (3, 12). All of these are still constructed by making use of an antibiotic resistance marker gene, which makes them not suitable for use in practice. Thus, the results of testing a human *eae* deletion mutant (8,10) and a rabbit secretion (*sep*) mutant (4a) as possible vaccine strains indicated that keeping the *eae* gene intact is necessary, as intimin is considered an important antigen for inducing a protective immune response.

It is thus an aim of the present invention to provide attenuated enteropathogenic *E. coli* strains which can be effectively used as a vaccine in mammals.

It is another aim of the present invention to provide a method for producing an attenuated mutant enteropathogenic *E. coli* strain which can be effectively used as a vaccine in mammals.

It is another aim of the present invention to provide a recombinant vector useful for producing an attenuated mutant enteropathogenic *E. coil* strain which can be effectively used as a vaccine in mammals.

It is another aim of the present invention to provide a vaccine composition against enteropathogenic *E. coli* infections in mammals.

It is another aim of the present invention to provide a method for *in vitro* differentiating between enteropathogenic *E. coli* field infected and vaccinated mammals.

The present inventors have demonstrated that deletions in the coding sequence of at least one of the genes in the LEE locus of EPEC strains results in an attenuation of the virulence of said strains.

The present invention relates to an attenuated non-human mammalian enteropathogenic *E. coli* (EPEC) strain comprising a deletion of an intimin encoding gene sequence wherein said deletion comprises the complete coding sequence or part of the coding sequence resulting in a frame shift mutation or resulting in a transcriptional or translational stop signal, further characterized in that said deletion fully eliminates (the expression of) a functional intimin protein.

Although deletion mutagenesis of *eae* gene sequences in human EPEC strains resulted in a strain that gave only slight protection against experimental infection, the present inventors surprisingly found that deletion of an *eae* gene sequence downstream of the suspected transmembrane anchor region of intimin in mammalian EPEC strains resulted in an effective protection against experimental infection.

The expression "attenuated strain" relates to a strain which is less virulent than the parental strain.

The expression "enteropathogenic E. coli (EPEC) strain" relates to a group of attaching and effacing (AE) *Escherichia coli* strains which are known as an important etiologic agent of human infant and animal diarrhea. Another important group of AE strains are the enterohemorrhagic E.coli strains (EHEC) which exhibit the similar unique virulence mechanism (27).

It should be understood that the expression "said deletion fully eliminates the expression of a functional intimin protein" relates to deletions of the coding sequences which can comprise the total coding sequence of the intimin gene, but can also comprise only part of the coding sequence resulting in a frame shift mutation or resulting in a transcriptional or translational stop signal. As a consequence, the intimin protein is not expressed in a functionally active form at the surface of the cell.

Therefore, the present invention also relates to an attenuated rabbit enteropathogenic *E. coli* (EPEC) strain comprising a deletion of an intimin encoding gene sequence with said strain being further characterized in that it induces an immune response when it is administered to a rabbit.

According to the invention, the attenuated mamalian non-human enteropathogenic *E. coli* (EPEC) strain is further characterised in that said strain contains fimbriae on its cell surface.

Fimbriae may be necessary to adhere and colonize the darmvilli and, as a consequence, to induce a protective immune response. On the other hand, an immune response against the main adhesines may be protective due to the prevention of this colonization.

The invention relates to non-human mammalian attenuated enteropathogenic *E. coli* (EPEC) strains. Preferred non-human mammalian strains are from rabbit, dog, cat, horse, goat, bovine, pig or poultry.

According to yet another preferred embodiment, the present invention relates to an attenuated rabbit enteropathogenic *E. coli* (EPEC) strain comprising a deletion of an intimin encoding gene sequence wherein said deletion fully eliminates a functional intimin protein and/or with said strain further characterized that it induces an immune response when it is administered to a mammal.

According to a further preferred embodiment, the present invention provides an attenuated rabbit enteropathogenic *E. coli* (EPEC) strain wherein said deletion consists of a deletion of an internal 248 basepair region of the *eae* gene. Said deletion should be such that it fully eliminates a functional intimin protein.

The present invention also provides attenuated rabbit enteropathogenic *E. coli* (EPEC) as deposited under No LMG P-18935 on April 22, 1999 in the Belgian Coordinated Collections of Microorganisms LMG collection. A tetracyclin-sensitive variant of this strain has been deposited under No LMG P-19461 on May 12, 2000 in the Belgian Coordinated Collections of Microorganisms LMG collection

According to another embodiment, the present invention relates to a recombinant vector comprising intimin encoding gene sequences in which a deletion is present, wherein said deletion fully eliminates the expression of functional intimin protein and wherein said deletion is one of the following: (i) a deletion downstream of the transmembrane anchor region of intimin, or (i) a deletion comprising part of the coding sequence resulting in a frame shift mutation or resulting in a transcriptional or translational stop signal, further characterized in that said intimin encoding gene sequence is from a non-human mammalian enteropathogenic E. coli (EPEC) strain.

More particularly, the present invention relates to a vector as defined above wherein said intimin encoding gene is the *eae* gene from a rabbit EPEC strain.

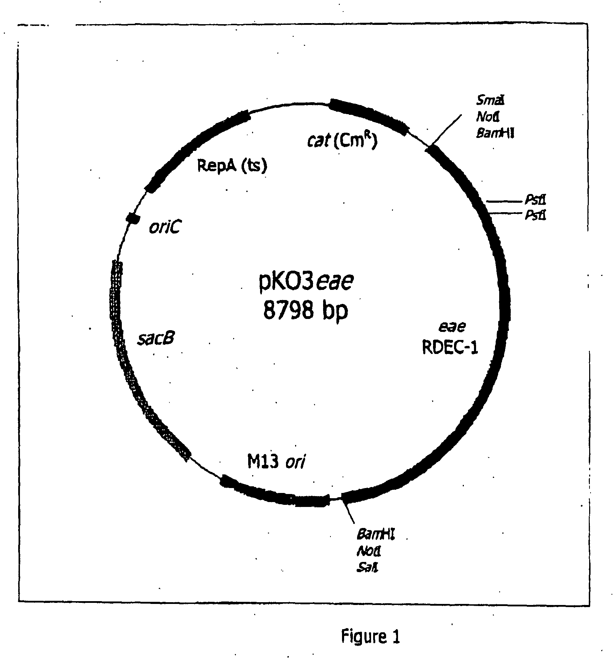

Even more particularly, the present invention relates to a vector as defined above termed pKO3Δ*eae* and characterized by a restriction map as shown in Figure 1.

According to another embodiment, the present invention relates to a method for producing a non-human attenuated mammalian enteropathogenic *E. coli* (EPEC) strain as defined above comprising the step of introducing an *eae* gene deletion in a wild-type EPEC strain by homologous recombination using a vector comprising a deletion of an intimin encoding gene sequence as defined above.

According to another embodiment, the present invention relates to a method for producing an attenuated rabbit enteropathogenic *E. coli* (EPEC) strain as defined above comprising the step of introducing an *eae* gene deletion in a wild-type EPEC strain by homologous recombination using a vector comprising a deletion of an intimin encoding gene sequence as defined above.

The methods of the invention are not limited to the production of attenuated rabbit enteropathogenic *E.coli* (EPEC) strains, but can be applied for the production of attenuated non-human enteropathogenic *E.coli* strains originating from other hosts, such as a dogs, cats, horses, goats, cattle, pig or poultry.

According to yet another embodiment, the present invention relates to an immunogenic composition comprising attenuated rabbit enteropathogenic *E. coli* (EPEC) strain comprising a deletion of an intimin encoding gene sequence as defined above.

According to another embodiment, the present invention relates to a live vaccine composition which provides protective immunity against an EPEC infection in a rabbit comprising a rabbit attenuated mammalian enteropathogenic *E. coli* (EPEC) strain comprising a deletion of an intimin encoding gene sequence wherein said deletion comprises the complete coding sequence or part of the coding sequence resulting in a frame shift mutation or resulting in a transcriptional or translational stop signal, further characterized in that said deletion fully eliminates the expression of a functional intimin protein.

According to a preferred embodiment, said mammalian strain is a non-human mammalian strain. As such, the present invention relates to a live vaccine composition which provides protective immunity against an EPEC infection in a rabbit comprising attenuated rabbit enteropathogenic *E. coli* (EPEC) strain comprising a deletion of an intimin encoding gene sequence as defined above.

If necessary, however, attenuated non-human mammalian enteropathogenic *E. coli* (EPEC) strain may also be administered by any other route known in the art to aid the treatment of enteropathogenic *E. coli* (EPEC) infections.

According to a preferred embodiment, said vaccine composition is an oral vaccine.

Administration of the attenuated enteropathogenic *E. coli* (EPEC) strains of the invention may be performed by adding any adjuvant or any other immune response enhancing agent known in the art.

According to another embodiment, the present invention relates to an in vitro method for differentiating between field infected and vaccinated individuals wherein said field infected individuals are *in vitro* diagnosed on the basis of the presence of intimin antibodies.

According to a preferred embodiment, the present invention relates to an in vitro method for differentiating between field infected and vaccinated individuals wherein the differentiation between wild type and mutant strain is made by using nudeic acid molecules (primers) represented by SEQ ID NOs 3 and 4 in an amplification reaction.

According to another embodiment, the present invention relates to the use of an attenuated rabbit enteropathogenic *E. coli* (EPEC) strain comprising a deletion of an intimin encoding gene sequence as defined above for the preparation of a vaccine wherein said deletion comprises the complete coding sequence on part of the coding sequence resulting in a frame shift mutation or resulting in a transcriptional or translational stop signal, further characterized in that said deletion fully eliminates the expression of a functional intimin protein.

The invention, now being generally described, will be more readily understood by reference to the following examples, which are included merely for purposes of illustration of certain aspects and embodiments of the present invention and are not intended to limit the invention.

### FIGURE LEGENDS

***Figure 1 :*** pKO3*eae* (8798 bp) results from the cloning of the *eae* sequence from RDEC-1 into the *Bam*HI restriction site of pKO3. Apart from the restriction sites for *Sma*I, *Not*I, *Bam*HI, *Eco*RI, *Pst*I and *Sal*I, the following features are shown : *cat* (chloramphenicol acetyltransferase), M13 *ori* (f1 (+) filamentous origin), *sac*B (*Bacillus subtilis sacB* gene), *ori*C, RepA (pSC101 tempereture sensitive). Cutting this vector with Pstl and re-ligating results in plasmid pKO3Δ*eae* having a deletion of the *eae* gene.
***Figure 2 :*** PCR products from REPEC 015 :H-Δ*eae* (lane 1), wild type 97/241.6 (lane 2), a negative control (water, lane 3) and a DNA size marker (λ DNA gel cut with *Pst*I) are separated on a gel (0,8% agarose in a tris-borate ethylene diamine tetra-acetate (TBE) buffer). The primers used (SEQ ID NO 3 and SEQ ID NO 4) align upstream and downstream the 248 bp deleted region in *Δeae*, resulting in a 350 bp product for an uncut *eae* (lane 2) and in a 102 bp product for the *Δeae* recombinant gene (lane 1).
***Figure 3 :*** The result of a Western blot from isolated membrane proteins detected by applying available polyclonal serum against rabbit EPEC (47) is shown. The technique used is described elsewhere (40, 47).
   Lane 1 shows the Biotinylated Broad Standard (Bio-Rad), lane 2 proteins from REPEC O15:H-Δ*eae* and lane 3 proteins from wild type strain 97/241.6. REPEC O15:H-Δ*eae* seems to have lost the intimin band (94kDa, arrow), while 97/241.6 still produces this band.
***Figure 4*** : Pane A shows the intimate and the effacing of microvilli by wild type strain 97/233.10 (3500X scanning electron microscope, SEM). Pane B shows identical effects observed after infection with wild type strain 97/241.6 (3500X). Pane C shows an overview of the same sample (900X) with a clear difference between uninfected and infected regions.
***Figure 5*** : A scanning electron microscope picture is shown taken after the infection of a rabbit with the attenuated REPEC 015:H-Δ*eae* strain (900X, SEM). No attachment of effacement by this strain is observed.
***Figure 6*** : Graphical presentation showing the daily feed uptake as measured and calculated three times a week. A significant difference is found between the non vaccinated and vaccinated/control group (confidence interval 95%).

### EXAMPLES

### Example 1. Target strain selection

Biotyping was performed on a collection of putative rabbit EPEC strains available in the inventors' laboratory, by using routine methods (30, 38). Motility, indol reaction en omithine decarboxylase activitity are tested on MIO tubes (GIBCO, Paisley, Scotland) following the manufacturer's specifications. Serotyping was performed by slide agglutination with specific antisera (31). Typing was repeated after safely storing the strains in a -70°C freezer. The presence of the locus of enterocyte effacement was detected by routine colony hybridization with non-radioactive probes A,B,C and D (22, 44). These probes cover the whole LEE genetic locus. Hybridization was performed at 65°C and Standard buffer was chosen as hybridization buffer (46). Based on these findings, two O15:H- strains were chosen as reference strains for the pathogenic O15:H- rabbit EPEC group, namely 97/223.10 and 97/241.6. These strains are available upon request from Centrum voor Onderzoek in Diergeneeskunde en Agrochemie, Groeselenberg 99, B-1180 Brussel (Ukkel), Belgium.

### Example 2. Construction and analysis of a mutant EPEC strains

### 1. Strain REPEC O15:H⁻ Δeae

The *eae* genes of 97/241.6, 97/223.10 and RDEC-1 were cloned by using standard techniques described in (44). Based on the published sequence of RDEC-1 *eae* (4), primers were chosen and PCR was performed on chromosomal DNA of the respective strains (primer up: 5' AAAAAAGGATCCAAAAATTACGCCGGAAGAT 3' (SEQ ID NO 1); primer down: 5' AAAAAATCTAGAAAAAGGTAAACKAGGTCTC 3' (SEQ ID NO 2); 10 cycles 15" 94°C, 30" 55,4°C, 2' 68°C followed by 20 cycles 15" 94°C, 30" 55,4°C, 2' 68°C + 20" each cycle). Expand™ polymerase (Boehringer Mannheim GmbH, Mannheim, Germany) was used and reactions were run on a Perkin Elmer GeneAmp 9600 Thermal Cycler. PCR products were cloned by using the pCR2.1-TOPO vector cloning system (Invitrogen, Groningen, The Netherlands). The RDEC-1 *eae* gene was cut out of the pCR2.1-TOPO plasmid with *Bam*HI and ligated in equalliy cut pKO3 (21), resulting in plasmid pKO3*eae* (Figure 1). Correct ligation was checked by restriction digest and get electrophoresis and by PCR (following the procedure described above, with recombinant plasmid as template DNA). An internal 248 basepair (bp) region of *eae* was removed by digesting pKO3*eae* with *Pst*I and self-ligating the plasmid, resulting in pKO3Δ*eae*. A PCR reaction test with primers flanking the deleted 248 bp region was designed (primer up 5' CCACTCAAAAAACTGTCTGTTG 3' (SEQ ID NO 3); primer down 5' TCCAGTGAACTACCGTCAAAG 3' (SEQ ID NO 4); 25 cycles 15" 94°C, 58,1°C, 72°C), making the detection of correctly ligated pKO3Δ*eae* easier. In case the 248 bp area was not cut out, the resulting product should be 350 bp and in case of a correct deletion 102 bp.

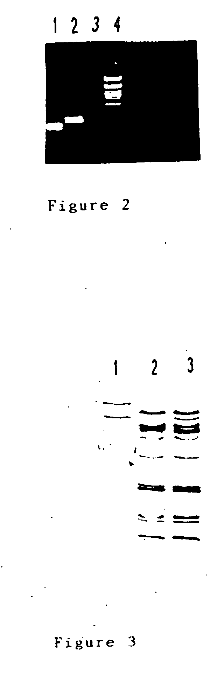

Plasmid pKO3 is a suicide plasmid with a temperature sensitive origin of replication *(ori)* and a saccharose sensitivity gene (*sacB*) from *Bacillus subtilis* as its mean feaures (21). This combination allowed it.to be used as a tool for site-specific mutagenesis by promoting two succeeding homologuous recombinations. The vector was originally designed as a helper tool to analyze unidentified open reading frames of *Escherichia coli*, also known as genomics research (21). The following strains isolated from various mammals were made electrocompetent following routine techniques (44) and were electroporated (conditions used: 200Ω, 25 F, 1.80kV, using a 0.1 cm cuvette in a Bio-Rad Gene Pulser): 97/96, 91/387, 95/148.2, 97/251, 97/44, 97/46.5, 98/33.1, 97/226, 97/224, 97/241.6, 97/223.10, 148KH96, 390KH97, 408KH97, 410KH97, 415KH97, 7KH98, 10KH98, 11KH98, 104KH99, 105KH99, 106KH99, 144KH99, 88KH00 and 155KH00. Electroporated strains were grown in SOC broth for 30 minutes in a shaking incubator and were streaked out on agar plates as described by (21). Double homologuous recombination did not succeed, but following the method described by (21) in which the vector pKO3Δ*eae* first cointegrates with *eae* in the chromosome and then undergoes a second recombination, did result in numerous colonies. The second recombination event resulted in either the intact eae gene or *Δeae*. Since the deleted region was situated close to the N terminal end of *eae* (405 bp from the start codon) and further from the C terminal region (2166 bp from the stop codon), a recombination leaving *Δeae* was less favourable. Possible mutant colonies were picked up and tested by the PCR detection test (see Figure 2) described above or by colony hybridization using the same conditions as described above but with the 248 bp region as a probe (made by performing PCR with the flanking primers on the wild type strains' chromosomes and by cutting the resulting product with *Pst*I, resulting in the deleted 248 bp region probe). An *E. coli* that lost the 248 bp region has been isolated, resulting in the mutant strain REPEC O15:H⁻ *Δeae* deposited under No. LMG P-18935 on April 22, 1999 in the Belgian Coordinated Collections of Microorganisms LMG collection.

### 2. Protein analysis of the mutant strain

Hypermmune serum against a O109:H+ strain, obtained in earlier described experiments (47), was used to visualize antigenic proteins of the REPEC O15:H⁻ *Δeae* and 97/241.6 strains by immunoblotting performed as described before (40). The band of 94 kilodalton (kDa) size which has been identified as intimin was no longer visible in the lane where proteins of REPEC O15:H⁻ *Δeae* were loaded (see Figure 3). This allowed the inventors to conclude that intimin was absent in the mutant strain, as expected.

### 3. Construction of the tetracycline sensitive strain REPEC O15:H⁻ Δeae Tc^{s}

A REPEC 015:H- *Δeae* tetracycline sensitive strain was obtained using Bochner selection plates (4b). Fusaric acid (2mg/ml was used as chelating agent. Approximately 106 cells were plated on a Bochner plate and subsequently incubated at 37°C for 24-36 hours. Colonies were picked, streaked for single colonies and plated again on a plate of Bochner medium. This procedure was repeated until a strain clearly sensitive to tetracycline was obtained. The resulting tetracycline sensitive strain REPEC O15:H⁻ *Δeae* Tc^{s} deposited under No. LMG P-19461 on May 12, 2000 in the Belgian Coordinated Collections of Microorganisms LMG collection

### Example 3. Pathogenic properties of mutant and non-mutant strains

Pathogenic properties of 97/241.6, 97/223.10 and REPEC O15:H⁻ *Δeae* strain were tested by performing an *in vivo* infection experiment. Two New Zealand White MDL (Minimum Disease Level) rabbits were tested per strain, two control animals were housed separately and were never infected. These rabbits were purchased from N.V. Verlabreed (Nevele, Belgium) a breeder with well known hygienic status. Their feed contained no coccidiostatics nor antibiotics. Control upon arrival showed that they were EPEC, *Clostridium spiroforme*, rotavirus and *Eimeria* free (37, 48). Experimental rooms were desinfected prior to use, as were the individual cages. Rabbits were fed with commercial food free from anticoccidiostatics and food uptake and daily weigth gain were followed regularly. Experimental infection was done by giving *per os* 1 ml of a cell suspension grown overnight and diluted in phosphate buffered saline (PBS) to 1.6 x 10⁴ CFU's per ml. One of two rabbits was euthanized 7 days after infection, the second 10 days after infection. Tissue samples from duodenum, jejunum, ileum and caecum were taken for light microscope and electron microscope study (36).

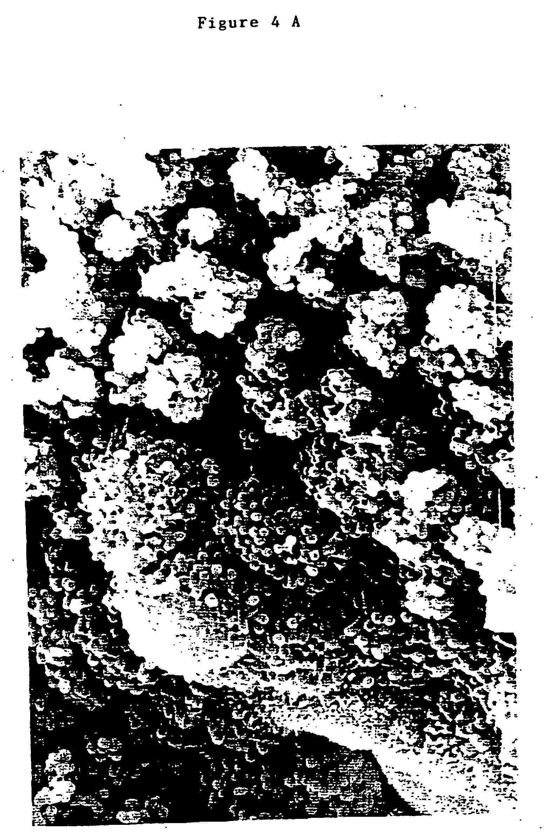

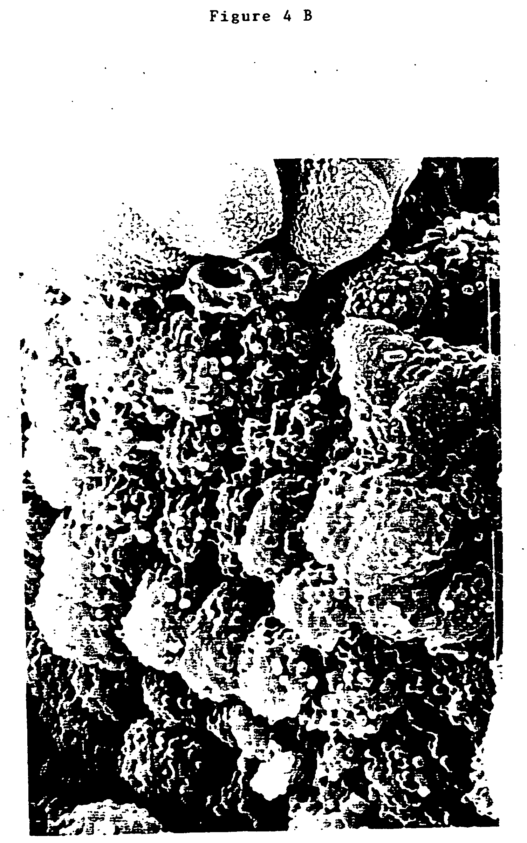

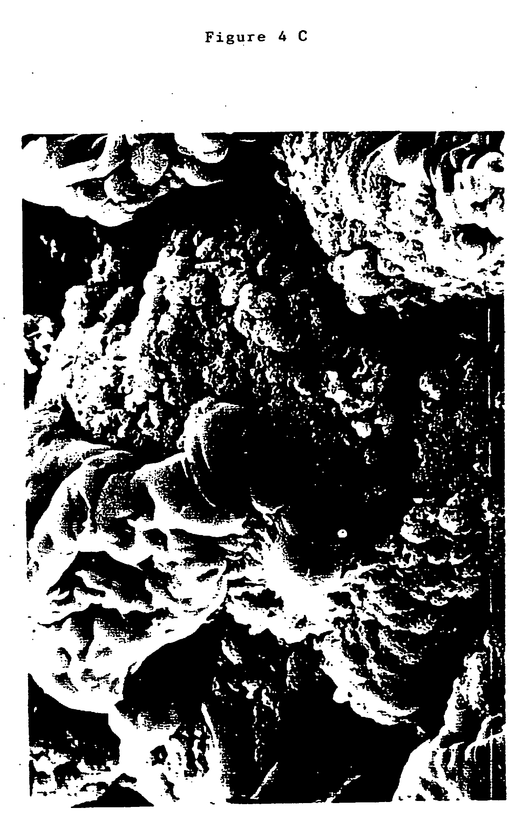

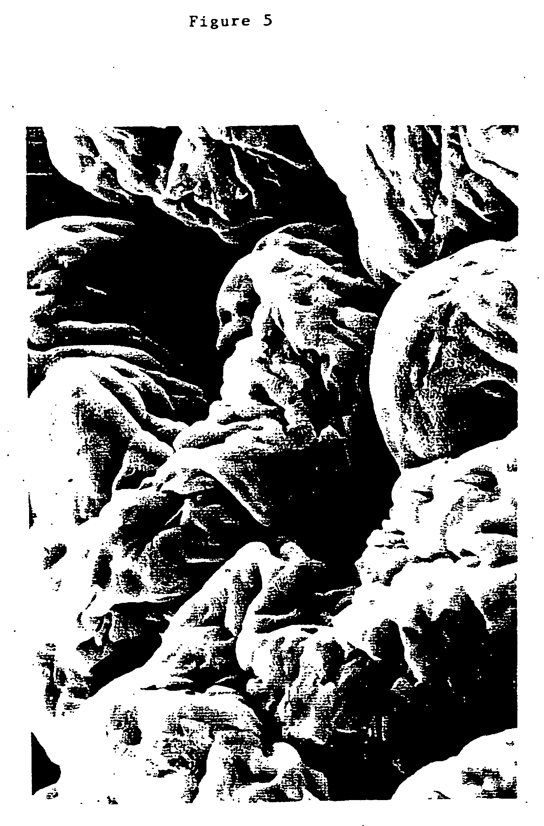

Clinical observations showed mild (97/223.10), light (97/241.6) and no diarrhea (control and REPEC O15:H⁻ Δ*eae*). Infection doses were rather low, because death caused by colibacillosis would interfere with clear histological observations using the microscope. Upon infection with 97/241.6 and 97/223.10, the feed conversion rate was clearly decreased, especially for the 97/223.10 infected rabbits. The feed conversion rate of REPEC O15:H⁻ *Δeae* infected rabbits was not negatively influenced compared to the controle rabbits. Histological studies showed that 97/223.10 attached intimately, pedestals were seen and microvilli were effaced, resulting in clearly destructed cells in surrounding regions. These effects were stronger visible in the caecum, but also detectable in the ileum (see Figure 4). Infection with 97/241.6 lead to identical observations. Infection with REPEC O15:H⁻ *Δeae* did however show that the strain was not attached intimately, but appeared concentrated in microcolonies in the lumen (see Figure 5). Tissue taken from control rabbits did not show attached organisms. Histological findings were observed by following the methods described in (36).

### Example 4. Adhesion of the mutant strain to the brush border of the darmvilli

Assuming that a certain persistence of the EPEC strain is necessary for a protective immune response to develop and hence, protect the host against a new infection, the present inventors made sure that, after deletion of the *eae* gene, the mutant is still capable to attach to enterocytes.

An *in vitro* test was performed. Darmvilli of a 6 weeks old New-Zealand rabbit were isolated using a technique described earlier (12a, 45a). The mutant strains were FITC-labelled by incubating 10⁸ microorganisms during 30 minutes with a solution of 0.5-mg FITC/1 ml 0.1 M Na₂CO₃. After washing the bacteria several times with PBS they were incubated during 1 hour with the separated darmvilli. A few but certain amount of bacteria adhered to the brush borders. Again B10 and DH5α were used as positive and negative control strains.

In addition, proof of the persistence of the mutant *in vivo* was obtained by plating daily faecal samples of rabbits, which were orally administered the mutant strain, on a SCS (Simmon citrate agar including 4% w/v sorbose and 250 ppm novobiocine) and G₂S (Gassner agar, yeast extract, Na₂S₂O₃.5H₂O, Fe²⁺-citrate and 25% novobiocine) agar. The mutant strains could be isolated for a period of more then 20 days.

### Example 5.

### 1. Immunoprotection by using the mutant strain in a vaccine

A vaccination experiment was performed to observe the immunoprotective features of REPEC O15:H⁻ *Δeae* against an infection with 97/223.10. 42 Rabbits were purchased, controlled, housed and followed as described above. They were divided in three groups with 14 rabbits each, in a way that the average weight of the three groups were highly similar. The first group was experimentally vaccinated *per os* with 1 ml of a PBS solution with 2.5 x 10⁵ CFU's/ml REPEC O15:H⁻ Δ*eae*. One week later the same group, together with the second group was experimentally infected with 97/223.10 (5.4 x 10⁴ CPU's /ml). The third group served as control group and was never infected with an EPEC strain.

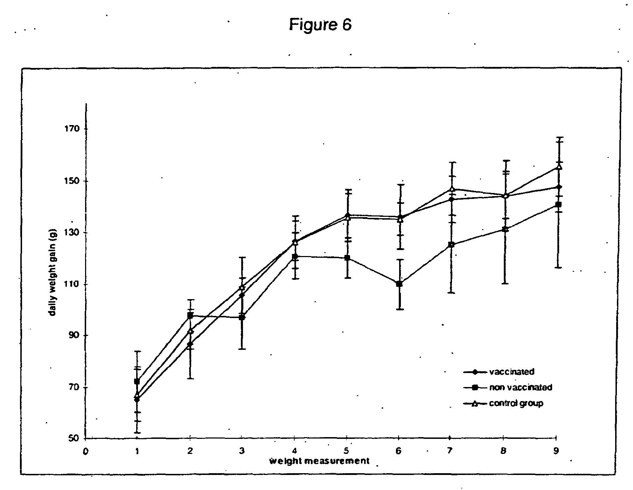

The group of non-vaccinated animals suffered more from diarrhea then the vaccinated rabbits. Even though the infection dose was low, one of the non-vaccinated animals died from colibacillosis as it was the only possibility upon autopsy. Non vaccinated animals showed a delay in weight gain compared to vaccinated and control animals. Also the daily feed uptake differed between vaccinated and non-vaccinated animals, as feed uptake of non-vaccinated animals is reduced to a significantly different level (alpha = 0.05) compared to control and vaccinated rabbits (see Figure 6). When feed conversions are calculated, no significant differences are found with the Student t-test between the three groups before infection with 97/223.10 (vaccinated to control: p=0.52; vaccinated to non-vaccinated: p=0.35; control to non-vaccinated: p=0.73). Following infection of group 1 and 2, feed conversions differ clearly for non-vaccinated and vaccinated rabbits (vaccinated to control: p=0.68; vaccinated to non-vaccinated: p=0.02; control to non-vaccinated: p=0.02). The feed conversion ratio of the non-vaccinated rabbits is significantly lower compared to the ratio's of vaccinated and control strains. No significant difference was found between vaccinated and control groups.

### 2. The obtained strains as a basis for further vaccine investigation

Further study of the strain learns us more about the signalling between the pathogenic organism and its host. Adhesion characteristics are studied *in vitro* on different cell lines. This, together with sequencing of the region surrounding the deleted 248 bp region gives more clarity about the precise features of REPEC 015:H⁻Δ*eae* and REPEC 015:H⁻Δeae Tc^{s}. The strains are tested for their precise immunogenic and protective role by following humoral and T-cell responses. Additional modifications are tested for their immunogenic effect.

### Example 6. Presence of fimbriae

Based on earlier published results a PCR was performed on chromosomal DNA of the respective strains to detect the in the rabbit prevalent adhesines, namely AF/R2 and RalE (3, 7, 7b, 40a). The test was positive for as well AF/R2 as RalE (primer up AF/R2: 5' GAAACTAATTGCTATTGCTG 3' (SEQ ID NO 5); primer down AF/R2: 5' ATGCGATTGAAATAGTTAGC 3' (SEQ ID NO 6); primer up RalE: 5' TTTGCCAAAATGAAACGCTT 3' (SEQ ID NO 7); primer down RalE: 5' AGCGCTTTTTGTTTACTTCC 3' (SEQ ID NO 8); 25 cycles 15" 94°C, 30" 53,1°C for AF/R2 and 30" 52.7°C for RalE, 2' 72°C). For most strains, the presence for EAF (enteroadherence factor) was tested as well. The results are listed below. (Table 1.)

**Table 1:**

| **Presence of fimbriae in some LEE-positive pathogenic *E coli*.** | | | | |
|---|---|---|---|---|
| *Name* | *Phenotype* | *Fimbriae* | | |
| | | AF/R2 | RalE | EAF |
| 97/223.10 | 3-/O15 | + | + | + |
| 97/241.6 | 3-/O15 | + | + | + |
| RDEC-1 | 3-/O15 | - | - | + |
| DH5α | 3-/O15 | - | - | NA |
| E2348/69 | 3-/O15 | - | - | NA |
| 97/204 | 3-/O15 | - | + | NA |
| 97/193.1 | 3-/O15 | + | + | + |
| 97/190.1 | 3-/O15 | + | + | + |
| 97/185.1 | 3-/O15 | + | + | + |
| 97/17.2 | 3-/O15 | + | + | + |
| 97/146 | 3-/O15 | + | + | + |
| 97/127 | 3-/O15 | + | + | + |
| 97/119.1 | 3-/O15 | + | + | NA |
| 97/101.1 | 3-/O15 | + | + | + |
| 96/261 | 3-/O15 | + | + | + |
| 95/148.2 | 3-/O15 | + | + | NA |
| 97/226 | 8+/O103 | + | - | + |
| 97/110.6 | 8+/O103 | + | + | + |
| B10 | 8+/O103 | + | + | + |
| 91/184 | 4+/O26 | - | - | + |
| B13 RDZ 113 | 4+/O26 | + | + | NA |
| B13 RDZ 112 | 4+/O26 | + | + | NA |
| B13 RDZ 112 | 4+/O26 | + | + | NA |
| B13 RDZ 106 | 4+/O26 | + | + | NA |
| NA = Not Available | | | | |

### REFERENCES

1. **Abe, A., U. Heczko, R. G. Hegele, and B. Brett Finlay.** 1998. Two Enteropathogenic Escherichia coli Type III Secreted Proteins, EspA and EspB, Are Virulence Factors. J Exp Med. **188**:1907-1916.
2. **Abe, A., B. Kenny, M. Stein, and B. B. Finlay.** 1997. Characterization of two virulence proteins secreted by rabbit enteropathogenic Escherichia coli, EspA and EspB, whose maximal expression is sensitive to host body temperature. Infect Immun. **65**:3547-55.
3. **Adams, L. M., C. P. Simmons, L. Rezmann, R. A. Strugnell, and R. M. Robins-Browne.** 1997. Identification and characterization of a K88- and CS31A-like operon of a rabbit enteropathogenic Escherichia coli strain which encodes fimbriae involved in the colonization of rabbit intestine. Infect Immun. **65**:5222-30.
4. **Agin, T. S., J. R. Cantey, E. C. Boedeker, and M. K. Wolf.** 1996. Characterization of the eaeA gene from rabbit enteropathogenic Escherichia coli strain RDEC-1 and comparison to other eaeA genes from bacteria that cause attaching-effacing lesions. FEMS Microbiol Lett. **144**:249-58.
4a. **Boury, M., K. Grange, J.P. Nougayrede, E. Oswald, J. De Rycke and A.** Milon. 1998. Mutants du Locus of Enterocyte Effacement de Escheridia coli entéropathogènes du lapin et vaccination orale contre la collibacillose O103., P. 65-68. 7iemes Joumées de la Récherche Cunicole en France. ITAVI, 28 rue du Rocher, 75008 Paris, Lyon, France.
4b. **Bochner, B. R., H. C. Huang, G. L. Schieven, and B. N. Ames.** 1980. Positive selection for loss of tetracycline resistance. J Bacteriol. **143**:926-33.
5. **Camguilhem, R., and A. Milon.** 1990. Protection of weaned rabbits against experimental Escherichia coli O103 intestinal infection by oral formalin-killed vaccine. Vet Microbiol. **21**:353-62.
6. **Cantey, J. R., and R. K. Blake.** 1977. Diarrhea due to Escherichia coli in the rabbit: a novel mechanism. J Infect Dis. **135**:454-62.
7. **Cantey, J. R., L. R. Inman, and R. K. Blake.** 1989. Production of diarrhea in the rabbit by a mutant of Escherichia coli (RDEC-1) that does not express adherence (AF/R1) pili. J Infect Dis. **160**:136-41.
7a. **Connell, H., W. Agace, M. Hedlund, P. Klemm, M. Shembri, and C. Svanborg.** 1996. Fimbriae-mediated adherence induces mucosal inflammation and bacterial clearance. Consequences for anti-adhesion therapy. Adv Exp Med Biol. **408**:73-80.
7b. **Chalareng, C., F. Pillien, M. Boury, C. Tasca, J. De Rycke, and A. Milon.** 1997. AF/R2 adhesin and cytopathic effect as virulence traits of diarrhea- inducing Escherichia coli O103 in European rabbit. Adv Exp Med Biol. **412**:269-71.
8. **Donnenberg, M. S., and J. B. Kaper.** 1991. Construction of an eae deletion mutant of enteropathogenic Escherichia coli by using a positive-selection suicide vector. Infect Immun. **59**:4310-7.
9. **Donnenberg, M. S., and J. B. Kaper.** 1992. Enteropathogenic Escherichia coli. Infect Immun. **60**:3953-61.
10. **Donnenberg, M. S., C. O. Tacket, S.P. James, G. Losonsky, J. P. Nataro, S.S. Wasserman, J. B. Kaper, and M. M. Levine.** 1993. Role of the *eae*A gene in experimental enteropathogenic Escherichia coli infection. J Clin Invest **92**:1412-7.
11. **Elliott, S. J., L. A. Wainwright, T. K. McDaniel, K. G. Jarvis, Y. K. Deng, L. C. Lai, B. P. McNamara, M. S. Donnenberg, and J. B. Kaper.** 1998. The complete sequence of the locus of enterocyte effacement (LEE) from enteropathogenic Escherichia coli E2348/69. Mol Microbiol. **28**:1-4.
12. **Fiederling, F., M. Boury, C. Petit, and A. Milon.** 1997. Adhesive factor/rabbit 2, a new fimbrial adhesin and a virulence factor from Escherichia coli O103, a serogroup enteropathogenic for rabbits. Infect Immun. **65**:847-51.
12a. **Girardeau, J. P. 1980.** A new in vitro technique for attachment to intestinal villi using enteropathogenic Escherichia coli. Ann Microbiol (Paris). **131B**:31-7.
13. **Giron, J. A., A. S. Ho, and G. K. Schoolnik.** 1991. An inducible bundle-forming pilus of enteropathogenic Escherichia coli. Science. **254**:710-3.
13a. **Hartland, E. L., M. Batchelor, R. M. Delahay, C. Hale, S. Matthews, G. Dougan, S. Knutton, I. Connerton, and G. Frankel.** 1999. Binding of intimin from enteropathogenic Escherichia coli to Tir and to host cells. Mol Microbiol. **32**:151-8.
14. **Hicks, S., G. Frankel, J. B. Kaper, G. Dougan, and A. D. Phillips.** 1998. Role of intimin and bundle-forming pili in enteropathogenic Escherichia coli adhesion to pediatric intestinal tissue in vitro. Infect Immun. **66**:1570-8.
15. **Jarvis, K. G., J. A. Giron, A. E. Jerse, T. K. McDaniel, M. S. Donnenberg, and J. B. Kaper.** 1995. Enteropathogenic Escherichia coli contains a putative type III secretion system necessary for the export of proteins involved in attaching and effacing lesion formation. Proc Natl Acad Sci U S A. **92**:7996-8000.
16. **Jerse, A. E., J. Yu, B. D. Tall, and J. B. Kaper.** 1990. A genetic locus of enteropathogenic Escherichia coli necessary for the production of attaching and effacing lesions on tissue culture cells. PNAS. **87**:7839-7843.
17. **Karaolis, D. K., T. K. McDaniel, J. B. Kaper, and E. C. Boedeker.** 1997. Cloning of the RDEC-1 locus of enterocyte effacement (LEE) and functional analysis of the phenotype on HEp-2 cells. Adv Exp Med Biol. **412**:241-5.
17a. **Kelly, G., S. Prasannan, S. Daniell, K. Fleming, G. Frankel, G. Dougan, I. Connerton, and S. Matthews.** 1999. Structure of the cell-adhesion fragment of intimin from enteropathogenic Escherichia coli. Nat Struct Biol. **6**:313-8.
18. **Kenny, B., R. DeVinney, M. Stein, D. J. Reinscheid, E. A. Frey, and B. B. Finlay.** 1997. Enteropathogenic E. coli (EPEC) transfers its receptor for intimate adherence into mammalian cells. Cell. **91**:511-20.
19. **Knutton, S., M. M. Baldini, J. B. Kaper, and A. S. McNeish.** 1987. Role of plasmid-encoded adherence factors in adhesion of enteropathogenic Escherichia coli to HEp-2 cells. Infect Immun. **55**:78-85.
20. **Knutton, S., I. Rosenshine, M. J. Pallen, I. Nisan, B. C. Neves, C. Bain, C. Wolff, G. Dougan, and G. Frankel.** 1998. A novel EspA-associated surface organelle of enteropathogenic Escherichia coli involved in protein translocation into epithelial cells. Embo J. **17**:2166-76.
21. **Link. A. J., D. Phillips, and G. M. Church.** 1997. Methods for generating precise deletions and insertions in the genome of wild-type Escherichia coli: application to open reading frame characterization: J Bacteriol. **179**:6228-37.
21a. **Marches, O., J. P. Nougayrede, S. Boullier, J. Mainil, G. Charlier, I. Raymond, P. Pohl, M. Boury, J. De Rycke, A. Milon, and E. Oswald.** 2000. Role of tir and intimin in the virulence of rabbit enteropathogenic escherichia coli serotype O103:H2. Infect Immun. **68**:2171-82.
22. **McDaniel, T. K., K. G. Jarvis, M. S. Donnenberg, and J. B. Kaper.** 1995. A genetic locus of enterocyte effacement conserved among diverse enterobacterial pathogens. Proc Natl Acad Sci U S A. **92**:1664-8.
23. **Milon, A., and R. Camguilhem.** 1989. Essais de protection de lapereaux sevrés contre l'entérite à Escherichia coli O103: vaccinations des mères avec un vaccin inactivé. Revue de Médecine Vétérinaire. **140**:389-395.
24. **Milon, A., and J. De Rycke.** 1997. Souches mutantes non pathogenes d'E. coli, leur procédé d'obtention et leurs utilisations., Institut National de la Propriété Industrielle, Paris., France.
25. **Milon, A., J. Esslinger, and R. Camguilhem.** 1992. Oral vaccination of weaned rabbits against enteropathogenic Escherichia coli-like E. coli O103 infection: use of heterologous strains harboring lipopolysaccharide or adhesin of pathogenic strains. Infect Immun. **60**:2702-9.
26. **Moon, H. W., S. C. Whipp, R. A. Argenzio, M. M. Levine, and R. A. Giannella.** 1983. Attaching and effacing activities of rabbit and human enteropathogenic Escherichia coli in pig and rabbit intestines. Infect Immun. **41**:1340-51.
27. **Nataro, J. P., and J. B. Kaper.** 1998. Diarrheagenic Escherichia coli [published erratum appears in Clin Microbiol Rev 1998 Apr,11(2):403]. Clin Microbiol Rev. **11**:142-201.
28. **Nisan, I., C. Wolff, E. Hanski, and I. Rosenshine.** 1998. Interaction of enteropathogenic Escherichia coli with host epithelial cells. Folia Microbiol. **43**:247-52.
29. **Nougayrede, J. P., O. Marches, M. Boury, J. Mainil, G. Charlier, P. Pohl, J. De Rycke, A. Milon, and E. Oswald.** 1999. The long-term cytoskeletal rearrangement induced by rabbit enteropathogenic Escherichia coli is Esp dependent but intimin independent [In Process Citation]. Mol Microbiol. **31**:19-30.
30. **Okerman, L., and L. A. Devriese.** 1985. Biotypes of enteropathogenic Escherichia coli strains from rabbits. J Clin Microbiol. **22**:955-8.
31. **Orskov, F., and I. Orskov.** 1984. Serotyping of Escherichia coli ., p. 43-112. *In* T. Bergan (ed.), Methods in Microbiology, vol. 14. Academic Press, Inc., London.
32. **Paton, A. W., P. A. Manning, M. C. Woodrow, and J. C. Paton.** 1998. Translocated intimin receptors (Tir) of shiga-toxigenic escherichia coli isolates belonging to serogroups O26, O111, and O157 react with sera from patients with hemolytic-uremic syndrome and exhibit marked sequence heterogeneity. Infect Immun. **66**:5580-6.
33. **Peeters, J., R. Geeroms, and B. Glorieux.** 1984. Experimental Escherichia coli enteropathy in weanling rabbits: clinical manifestations and pathological findings. Journal of Comparative Pathology. **94**:521-528.
33a. **Peelers, J. E., P. Phol, L. Okerman and L. A. Devriese.** 1984. Pathogenic properties of Escheridia coli strains isolated from diarrheie commercial rabbits. J Clin Microbiol **20**:34-9.
34. **Peeters, J. E.** 1994. E. coli infections in animals, p. 261-283. *In* G. C.L. (ed.), Escherichia coli in domestic animals and humans. CAB International, Wallingford, UK.
35. **Peeters, J. E., G. J. Charlier, and P. H. Halen.** 1984. Pathogenicity of attaching effacing enteropathogenic Escherichia coli isolated from diarrheic suckling and weanling rabbits for newborn rabbits. Infect Immun. **46**:690-6.
36. **Peeters, J. E., G. J. Charlier, and R. Raeymaekers.** 1985. Scanning and transmission electron microscopy of attaching effacing Escherichia coli in weanling rabbits. Vet Pathol. **22**:54-9.
37. **Peeters, J. E., R. Geeroms, R. J. Carman, and T. D. Wilkins.** 1986. Significance of Clostridium spiroforme in the enteritis-complex of commercial rabbits. Vet Microbiol. **12**:25-31.
38. **Peeters, J. E., R. Geeroms, and F. Orskov.** 1988. Biotype, serotype, and pathogenicity of attaching and effacing enteropathogenic Escherichia coli strains isolated from diarrheic commercial rabbits. Infect Immun. **56**:1442-8.
39. **Peeters, J. E., P. Pohl, and G. Charlier.** 1984. Infectious agents associated with diarrhoea in commercial rabbits: a field study. Ann Rech Vet. **15**:335-40.
40. **Peeters, J. E., I. Villacorta, E. Vanopdenbosch, D. Vandergheynst, M. Naciri, E. Ares-Mazas, and P. Yvore.** 1992. Cryptosporidium parvum in calves: kinetics and immunoblot analysis of specific serum and local antibody responses (immunoglobulin A [IgA], IgG, and IgM) after natural and experimental infections. Infect Immun. **60**:2309-16.
40a. **Pillien, F., C. Chalareng, M. Boury, C. Tasca, J. de Rycke, and A. Milon.** 1996. Role of Adhesive Factor/Rabbit 2 in experimental enteropathogenic Escherichia coli O103 diarrhea of weaned rabbit. Vet Microbiol. **50**:105-15.
41. **Pohl, P. H., J. E. Peeters, E. R. Jacquemin, P. F. Lintermans, and J. G. Mainil.** 1993. Identification of eae sequences in enteropathogenic Escherichia coli strains from rabbits. Infect Immun. **61**:2203-6.
42. **Rosenshine, I.** 1998. Species specificity and tissue tropism of EPEC and related pathogens. Trends Microbiol. **6**:388.
43. **Rosenshine, I., S. Ruschkowski, M. Stein, D. J. Reinscheid, S. D. Mills, and B. B. Finlay.** 1996. A pathogenic bacterium triggers epithelial signals to form a functional bacterial receptor that mediates actin pseudopod formation. Embo J. **15**:2613-24.
44. **Sambrook, J., E. F. Fritsch, and T. Maniatis.** 1989. Molecular cloning : a laboratory manual, 2nd ed. Cold Spring Harbor Laboratory, Cold Spring Harbor, N.Y.
45. **Taylor, K. A., C. B. O'Connell, P. W. Luther, and M. S. Donnenberg.** 1998. The EspB protein of enteropathogenic escherichia coli is targeted to the cytoplasm of infected HeLa cells [In Process Citation]. Infect Immun. **66**:5501-7.
45a. **Van den Broeck, W., E. Cox, B. Oudega, and B. M. Goddeeris.** 2000. The F4 fimbrial antigen of Escherichia coli and its receptors . Vet Microbiol. **71**:223-44.
46. **van Miltenburg, R., B. Ruger, S. Grunewald-Janho, M. Leons, and C. Schroder (ed.).** 1995. The DIG System User's Guide for Filter Hybridization. Boehirnger Mannheim GmbH, Mannheim.
47. **Vandekerchove, D., and J. Peeters.** 1998. Detection of specific antibodies against strains of enteropathogenic Escherichia coli (EPEC) in the rabbit by an ELISA using 94-kilodalton proteins. World Rabbit Science. **6**:303-310.
48. **Vanopdenbosch, E.** 1980. Immunodiffusion test for the detection of rotavirus antigen in faecal material and gut homogenates. Current Topics in Veterinary Medicine and animal Science. **13**:118-120.
49. **Wolff, C., I. Nisan, E. Hanski, G. Frankel, and I. Rosenshine.** 1998. Protein translocation into host epithelial cells by infecting enteropathogenic Escherichia coli. Mol Microbiol. **28**:143-55.

## Claims

1. An attenuated non-human mammalian enteropathogenic *E. coli* (EPEC) strain comprising a deletion of an intimin encoding gene sequence wherein said deletion comprises the complete coding sequence or part of the coding sequence resulting in a frame shift mutation or resulting in a transcriptional or translational stop signal, further **characterized in that** said deletion fully eliminates the expression of a functional intimin protein.

2. An attenuated rabbit enteropathogenic *E. coli* (EPEC) strain comprising a deletion of an intimin encoding gene sequence according to claim 1 with said strain being further **characterized in that** it induces an immune response when it is administered to a rabbit.

3. A strain according to claim 1 or 2 further **characterised in that** said strain contains fimbriae on its cell surface.

4. A strain according to claim 2 or 3 chosen from the strains as deposited under No LMG P-18935 on April 22, 1999 and No LMG P-19461 on May 12, 2000 in the Belgian Coordinated Collections of Microorganisms LMG collection.

5. A recombinant vector comprising intimin encoding gene sequences in which a deletion is present, wherein said deletion fully eliminates the expression of a functional intimin protein and wherein said deletion is one of the following: (i) a deletion downstream of the transmembrane anchor region of intimin, or (ii) a deletion comprising part of the coding sequence resulting in a frame shift mutation or resulting in a transcriptional or translational stop signal, further **characterized in that** said intimin encoding gene sequence is from a non-human mammalian enteropathogenic *E. coli* (EPEC) strain.

6. A vector according to claim 5 wherein said intimin encoding gene is the eae gene from a rabbit EPEC strain.

7. A method for producing a non-human mammalian enteropathogenic *E. coli* (EPEC) strain as defined in any of claims 1 to 4 comprising the step of introducing an *eae* gene deletion in a wild-type EPEC strain by homologous recombination using a vector according to any of claims 6 or 7.

8. An immunogenic composition comprising an attenuated rabbit EPEC strain of any of claims 2 to 4.

9. A vaccine composition which provides protective immunity against an EPEC infection in a rabbit comprising an rabbit enteropathogenic *E. coli* (EPEC) strain comprising a deletion of an intimin encoding gene sequence wherein said deletion comprises the complete coding sequence or part of the coding sequence resulting in a frame shift mutation or resulting in a transcriptional or translational stop signal, further **characterized in that** said deletion fully eliminates the expression of a functional intimin protein.

10. A vaccine composition according to claim 9 which is an oral vaccine.

11. An in vitro method for differentiating between field infected and vaccinated individuals wherein said field infected individuals are *in vitro* diagnosed on the basis of the presence of intimin antibodies.

12. An in vitro method for differentiating between field infected and vaccinated individuals wherein the differentiation between wild type and mutant strain is made by using primers represented by SEQ ID NOs 3 and 4 in an amplification reaction.

13. Use of an attenuated rabbit enteropathogenic *E. coli* (EPEC) strain comprising a deletion of an intimin encoding gene sequence for the preparation of a vaccine wherein said deletion comprises the complete coding sequence or part of the coding sequence resulting in a frame shift mutation or resulting in a transcriptional or translational stop signal, further **characterized in that** said deletion fully eliminates the expression of a functional intimin protein.

## Patentansprüche

1. Attenuierter nicht-menschlicher Säugetier-enteropathogener *E. coli* (EPEC)-Stamm, der eine Deletion einer Intimin codierenden Gensequenz enthält, wobei die Deletion die gesamte codierende Sequenz oder einen Teil der codierenden Sequenz umfasst, resultierend in einer Leseraster-Mutation oder resultierend in einem transkriptionalen oder translationalen Stoppsignal, dadurch weiter **gekennzeichnet, dass** die Deletion die Expression eines funktionellen Intimin-Proteins vollständig beseitigt.

2. Attenuierter Kaninchen-enteropathogener *E. coli* (EPEC)-Stamm, umfassend eine Deletion einer Intimin codierenden Gensequenz gemäß Anspruch 1, wobei der Stamm weiter **dadurch gekennzeichnet ist, dass** er eine Immunantwort induziert, wenn er einem Kaninchen verabreicht wird.

3. Stamm gemäß Anspruch 1 oder 2, dadurch weiter **gekennzeichnet, dass** der Stamm Fimbriae auf seiner Zelloberfläche enthält.

4. Stamm gemäß Anspruch 2 oder 3, ausgewählt aus den Stämmen, die unter Nr. LMG P-18935 am 22. April 1999 und Nr. LMG P-19461 am 12. Mai 2000 in der Sammlung der Belgischen Koordinierten Sammlung von Mikroorganismen LMG hinterlegt wurden.

5. Rekombinanter Vektor, umfassend Intimin codierende Gensequenzen, in denen eine Deletion vorhanden ist, wobei die Deletion die Expression eines funktionellen Intimin-Proteins vollständig beseitigt und wobei die Deletion eine der folgenden ist: (i) eine Deletion stromabwärts der transmembranen Ankerregion des Intimin, oder (ii) eine Deletion, die einen Teil der codierenden Sequenz umfasst, resultierend in einer Leseraster-Mutation oder resultierend in einem transkriptionalen oder translationalen Stoppsignal, dadurch weiter **gekennzeichnet, dass** die Intimin codierende Gensequenz von einem nicht-menschlichen Säugetier-enteropathogenen *E. coli* (EPEC)-Stamm stammt.

6. Vektor gemäß Anspruch 5, wobei das Intimin codierende Gen das *eae*-Gen von einem Kaninchen-EPEC-Stamm ist.

7. Verfahren zur Herstellung eines nicht-menschlichen Säugetier-enteropathogenen *E. coli* (EPEC)-Stamms, wie in einem der Ansprüche 1 bis 4 definiert, umfassend den Schritt des Einführens einer *eae*-Gendeletion in einen Wildtyp-EPEC-Stamm durch homologe Rekombination unter Verwendung eines Vektors gemäß einem der Ansprüche 6 oder 7.

8. Immunogene Zusammensetzung, umfassend einen attenuierten Kaninchen-EPEC-Stamm nach einem der Ansprüche 2 bis 4.

9. Impfstoff-Zusammensetzung, die eine schützende Immunität gegen eine EPEC-Infektion in Kaninchen bereitstellt, umfassend einen Kaninchen-enteropathogenen *E. coli* (EPEC)-Stamm, der eine Deletion einer Intimin codierenden Gensequenz umfasst, wobei die Deletion die gesamte codierende Sequenz oder einen Teil der codierenden Sequenz umfassen, resultierend in einer Leseraster-Mutation oder resultierend in einem transkriptionalen oder translationalen Stoppsignal, weiter **dadurch gekennzeichnet, dass** die Deletion die Expression eines funktionellen Intimin-Proteins vollständig beseitigt.

10. Impfstoff-Zusammensetzung gemäß Anspruch 9, die ein oraler Impfstoff ist.

11. *In vitro*-Verfahren zur Differenzierung zwischen feldinfizierten und geimpften Individuen, wobei die feldinfizierten Individuen auf der Basis der Anwesenheit von Intimin-Antikörpern *in vitro* diagnostiziert werden.

12. *In vitro*-Verfahren zur Differenzierung zwischen feldinfizierten und geimpften Individuen, wobei die Differenzierung zwischen Wildtyp- und mutiertem Stamm dadurch gemacht wird, dass Primer, dargestellt durch die SEQ ID Nrn. 3 und 4, in einer Amplifikationsreaktion verwendet werden.

13. Verwendung eines attenuierten Kaninchen-enteropathogenen *E. coli* (EPEC)-Stamms, umfassend eine Deletion einer Intimin codierenden Gensequenz für die Herstellung eines Impfstoffs, wobei die Deletion die gesamte codierende Sequenz oder einen Teil der codierenden Sequenz umfasst, resultierend in einer Leseraster-Mutation oder resultierend in einem transkriptionalen oder translationalen Stoppsignal, weiter **dadurch gekennzeichnet, dass** die Deletion die Expression eines funktionellen Intimin-Proteins vollständig beseitigt.

## Revendications

1. Souche atténuée d'E. *coli* entéropathogène de mammifère non humain (ECEP) comprenant une délétion de la séquence d'un gène codant pour une intimine, ladite délétion comprenant la séquence codante complète ou une partie de la séquence codante résultant en une mutation responsable d'un décalage du cadre de lecture ou résultant en un signal d'arrêt de la transcription ou de la traduction, **caractérisée en outre en ce que** ladite délétion supprime complètement l'expression d'une protéine intimine fonctionnelle.

2. Souche atténuée d'E. *coli* entéropathogène pour le lapin (ECEP) comprenant une délétion de la séquence d'un gène codant pour une intimine selon la revendication 1, ladite souche étant en outre **caractérisée en ce qu'**elle induit une réponse immune quand elle est administrée à un lapin.

3. Souche selon la revendication 1 ou 2, **caractérisée en outre en ce que** ladite souche contient des fimbriae sur sa surface cellulaire.

4. Souche selon la revendication 2 ou 3 choisie parmi les souches déposées sous le numéro No LMG P-18935 le 22 avril 1999 et No LMG P-19461 le 12 mai 2000 dans la collection Belgian Coordinated Collections of Microorganisms LMG.

5. Vecteur recombinant comprenant des séquences de gène codant pour l'intimine dans lesquelles une délétion est présente, ladite délétion supprimant complètement l'expression d'une protéine intimine fonctionnelle et ladite délétion étant l'une des suivantes : (i) une délétion en aval de la région d'ancrage transmembranaire de l'intimine, ou (ii) une délétion comprenant une partie de la séquence codante résultant en une mutation responsable d'un décalage du cadre de lecture ou résultant en un signal d'arrêt de la transcription ou de la traduction, **caractérisé en outre en ce que** ladite séquence du gène codant pour l'intimine est issue d'une souche d'E. *coli* entéropathogène de mammifère non humain (ECEP).

6. Vecteur selon la revendication 5, dans lequel ledit gène codant pour l'intimine est un gène eae issu d'une souche ECEP pour le lapin.

7. Méthode de production d'une souche d'E. *coli* entéropathogène de mammifère non humain (ECEP) telle que définie dans l'une quelconque des revendications 1 à 4 comprenant l'étape d'introduction d'une délétion de gène eae dans une souche ECEP sauvage par recombinaison homologue à l'aide d'un vecteur selon l'une des revendications 6 ou 7.

8. Composition immunogène comprenant une souche ECEP de lapin atténuée selon l'une des revendications 2 à 4.

9. Composition de vaccin qui confère une immunité protectrice vis-à-vis d'une infection par ECEP chez un lapin comprenant une souche d'E. *coli* entéropathogène de lapin (ECEP) comprenant une délétion d'une séquence d'un gène codant pour une intimine, ladite délétion comprenant la séquence codante complète ou une partie de la séquence codante résultant en une mutation responsable d'un décalage du cadre de lecture ou résultant en un signal d'arrêt de la transcription ou de la traduction, **caractérisée en outre en ce que** ladite délétion supprime complètement l'expression d'une protéine intimine fonctionnelle.

10. Composition de vaccin selon la revendication 9 qui est vaccin oral.

11. Méthode de différentiation *in vitro* entre des individus infectés en champs et des individus vaccinés, selon laquelle lesdits individus infectés en champs sont diagnostiqués *in vitro* sur la base de la présence d'anticorps contre l'intimine.

12. Méthode de différentiation *in vitro* entre des individus infectés en champs et des individus vaccinés, selon laquelle la différentiation entre les souches sauvages et mutantes est réalisée grâce à des amorces représentées par les séquences SEQ ID NOs 3 et 4 dans une réaction d'amplification.

13. Utilisation d'une souche atténuée d'E. *coli* entéropathogène de lapin (ECEP) comprenant une délétion d'une séquence d'un gène codant pour une intimine, pour la préparation d'un vaccin, ladite délétion comprenant la séquence codante complète ou une partie de la séquence codante résultant en une mutation responsable d'un décalage du cadre de lecture ou résultant en un signal d'arrêt de la transcription ou de la traduction, **caractérisée en outre en ce que** ladite délétion supprime complètement l'expression d'une protéine intimine fonctionnelle.
